Europäisches Patentamt

European Patent Office

Office européen des brevets

(19)

(11) Publication number: **0 368 232**
**A1**

# (12) EUROPEAN PATENT APPLICATION

(21) Application number: **89120582.5**

(22) Date of filing: **09.10.87**

(51) Int. Cl.5: **A61K 45/06, A61K 37/54,**
**//(A61K37/54,31:44,31:415)**

This application was filed on 07 - 11 - 1989 as a divisional application to the application mentioned under INID code 60.

(30) Priority: **09.10.86 US 917122**

(43) Date of publication of application:
**16.05.90 Bulletin 90/20**

(60) Publication number of the earlier application in accordance with Art.76 EPC: **0 265 129**

(84) Designated Contracting States:
**AT BE CH DE ES FR GB GR IT LI LU NL SE**

(71) Applicant: **SMITHKLINE BECKMAN CORPORATION**
**One Franklin Plaza P O Box 7929**
**Philadelphia Pennsylvania 19103(US)**

(72) Inventor: **Tsuneo, Fujita**
**512 Britton Drive**
**King of Prussia Pennsylvania 19406(US)**
Inventor: **Shebuski, Ronald John**
**231A Geryville PK**
**Pennsburg PA 18073(US)**

(74) Representative: **Giddings, Peter John, Dr. et al**
**Smith Kline & French Laboratories Ltd.**
**Corporate Patents Mundells**
**Welwyn Garden City Hertfordshire AL7 1EY(GB)**

(54) **Thrombolytic therapy.**

(57) An improved composition for effecting fibrinolysis and reperfusion following thrombotic occlusion of coronary vessels comprising a plasminogen activator, a thromboxane receptor antagonist and a pharmaceutically acceptable carrier.

EP 0 368 232 A1

## Thrombolytic Therapy

Field of the Invention

This invention relates to the field of cardiovascular pharmacclogy and, more specifically, to plasminogen activators and use thereof to effect fibrinolysis and reperfusion following thrombotic occlusion of coronary or other blood vessels.

Background of the Invention

Available therapies for reperfusion of occluded blood vessels include intravenous or intracoronary administration of a plasminogen activator, i.e, thrombolytic therapy. Predominant among plasminogen activators used in thrombolytic therapy are streptokinase (SK), of bacterial origin, and urokinase (UK) and tissue plasminogen activator (tPA), both of human origin.

tPA offers several advantages over SK and UK. These include lack of immunogenicity, a potential problem in the case of SK, and fibrin binding specificity, a characteristic which results in reduced systemic fibrinolysis.

Thrombolytic therapy at best, however, even with tPA, can only re-establish integrade flow in an infarcted artery. Thrombolytic therapy will not reverse the factors responsible for initiation of the thrombus such as advanced atherosclerotic plaques, intimal rupture, enhanced platelet adherence and aggregability or coronary spasm. Patients with a residual stenosis of greater than 80%, after successful recanalization with tPA, are at an extremely high risk of rethrombosis even in the presence of high dose heparin. See Gold et al., Circ. 73:347 (1986). In addition to persistent reocclusion, systemic bleeding can be associated with administration of high doses of tPA.

Thromboxane $A_2$ ($TXA_2$) is an arachidonate metabolite which is synthesized primarily in blood platelets. See Hamberg et al., Proc. Natl. Acad. Sci. USA 72:2994 (1975); Moncada et al., Pharmacol. Rev. 30:293 (1979). This agent has potent platelet aggregating and vasoconstrictor properties and has been the subject of much interest. Thromboxane synthase inhibitors have no known fibrinolytic effects but are specific inhibitors of the platelet enzyme thromboxane synthase, which converts the arachidonate-induced prostaglandin endoperoxide, $PGH_2$, to thromboxane $A_2$. Thromboxane synthase inhibitors have been demonstrated to be efficacious in inhibiting platelet aggregation in various animal models of platelet activation. See, Schumacher et al., J. Pharmacol. Exp. Ther. 277:719 (1983), and Lindsay et al., Thromb. Res. 43:127 (1986). Aiken et al., J. Pharmacol. Exp. Ther. 219:299 (1981) demonstrated that endogenous prostacyclin contributed to the efficacy of thromboxane synthase inhibition via diversion or "steal" of platelet-derived endoperoxides to form prostacyclin.

Schumacher et al., J.Cardiol.Pharmacol. 7:739 (1985), report that prostacyclin augments streptokinase-induced coronary thrombolysis in the dog.

Gallas et al., Fed. Proc. 45:96 (1986), reports that CGS 13080, a thromboxane synthase inhibitor, inhibits reocclusion following streptokinase-induced reperfusion after thrombotic occlusion of the circumflex coronary artery in a dog model.

It is an object of this invention to provide a thrombolytic therapy comprising internally administering a plasminogen activator and an agent which lowers the effective plasminogen activator dose requirement and which results in a lower incidence of reocclusion. It is also an object of this invention to provide a plasminogen activator composition comprising tPA which is effective at lower doses of tPA. It is a further object to provide such composition which is subject to a lower incidence of reocclusion.

Summary of the Invention

The invention lies in the discovery that the effective dose of a plasminogen activator in thrombolytic therapy, and the incidence of reocclusion associated therewith, can both be lowered by administration of a thromboxane receptor antagonist.

Specifically, this invention is a thrombolytic composition for parenteral administration to effect thrombolysis and reperfusion of a thrombotic occlusion in a mammal which comprises a plasminogen activator, a thromboxane receptor antagonist and a pharmaceutically acceptable carrier.

In another aspect, the invention is a method for effecting thrombolysis and reperfusion of a thrombotic occlusion in a mammal which comprises internally administering to the mammal an effective amount of a thromboxane receptor antagonist and an effective amount of a plasminogen activator.

In yet another aspect, the invention is a kit for use in a method for effecting thrombolysis and reperfusion of a thrombotic occlusion in a mammal which comprises, in one container, a thromboxane receptor antagonist in a second container, a plasminogen activator.

These and other aspects fully described below are considered to be further aspects of the same invention.


Detailed Description of the Invention


It has now been found that by use of a thromboxane receptor antagonist, the effective thrombolytic dose of a plasminogen activator is lowered significantly, the time to reperfusion is decreased and the incidence of reocclusion is reduced. Although the invention is not limited to a particular mechanism of action, this effect is believed to result from a desirable shift in a dynamic equilibrium of fibrinolysis induced by the plasminogen activator and platelet aggregation induced by the plasminogen activator and by other factors, including thromboxane.

Thromboxane receptor antagonists include:

EP045 and EP092 [Armstrong et al., Br. J. Pharmacol. 84:595 (1985)];

4-[2-(benzenesulfonamido)ethyl]phenoxyacetic acid (BM 13,177) [Patscheke et al., Thromb. Res. 33:277 (1984); U.S. 4,443,377];

4-[2-(phenylsulfonylamino)ethyl]phenoxyacetic acid (BM 13,505) [Stegmeier et al., Proc. EDTA-ERA 22: 1012 (1985) U.S. 4,258,058];

N,N'-[7-(3-chlorobenzene aminosulfonyl)-1,2,3,4-tetrahydroisoquinolyl]disulfonamide (SKF 88046) [Weichman et al., Prostaglandins Leukotrienes Med 15:167 (1984);

5029548 [Ogletree et al., J. Pharmacol. Exp. Ther. 234:435 (1985)];

AH23848 [Brittain et al., Circ. 72:1208 (1985)]; 13APA [LeBreton et al., Proc. Natl. Acad. Sci. U.S.A. 76:4097 (1979)] and,

ONO3708 [Kutsura et al., Adv. Pros. Thromb. LK. Res. 11:351 (1983)].

The invention further comprises the use of a thromboxane receptor antagonist as an adjunct to thrombolytic therapy, for treatment of a mammal, especially a human, in need thereof. Indications for such therapy include myocardial infarction, deep vein thrombosis, pulmonary embolism, stroke and other infarct-related disorders. The thromboxane antagonist is administered just prior to, at the same time as, or just following parenteral administration of a plasminogen activator.

Useful plasminogen activators include any pharmaceutically acceptable agent that activates plasminogen, such as streptokinase (SK), urokinase (UK) and tissue plasminogen activator (tPA), as well as related fibrinolytics such as acylated plasminogen streptokinase activator complex, prourokinase, single chain urokinase (SCUPA), antibody-bound plasminogen activators and hybrid tPA-UK proteins and other so-called "third generation" thrombolytics. Preferably, a plasminogen activator which has affinity for fibrin, as in the case of UK and tPA, is employed. tPA has a higher affinity for fibrin than does UK. tPA is the most preferred plasminogen activator for use in this invention.

The plasminogen activator used in this invention can be derived by synthesis or it can be derived biologically from tissue, from natural cell culture (e.g. Bowes melanoma cells for tPA and Calu cells for UK) or from recombinant bacteria, yeast or cell culture, or by a combination of biological and synthetic techniques. tPA includes variants of tPA which have the fibrin binding functions of tPA and plasminogen activating activity. Such variants include proteins differing by one or a few amino acids as well as variants having a different glycosylation pattern or carbohydrate composition or lacking any glycosyl moieties. See, e.g. Robinson, WO84-01786 and Rosa et al., WO86-01538. Fusions of the fibrin binding functions of tPA with other protease functions are included as such molecules have substantially the same biological properties of tPA. SK is typically prepared by fermentation of producing Streptococci in accordance with known procedures or can be expressed in recombinant cell culture. See, e.g. EP-A-151,337.

For use in the method of the invention, the plasminogen activator can be formulated with or without the thromboxane antagonist. The plasminogen activator is formulated in accordance with known procedures. Useful formulations of tPA are disclosed, for example, in co-pending U.S. patent application Serial No.

890,432 which is incorporated herein by reference. Useful formulations of UK are disclosed, for example, in EP-A-92,182.

The platelet thromboxane receptor antagonist, is formulated for oral or parenteral, e.g. intramuscular or intravenous, administration and administered so as to provide an amount which potentiates the thrombolytic activity of the plasminogen activator and which inhibits reocculusion. Such amount inhibits thromboxane-induced platelet aggregation at the site of the occlusion at least during the period of thrombolytic therapy, without causing significant adverse side effects.

In the case of a thromboxane receptor antagonist, the potentiation is recognized as an increase in the rate of reperfusion as compared with that observed with a plasminogen activator along, thus permitting use of a lower dose of plasminogen activator per patient treatment.

It may prove desirable to continue treatment with the inhibitor to maximize inhibition of post-therapy reocclusion for up to 72 hours. Oral maintenance therapy can be conducted, e.g. for six months. The formulation and administration of the antagonist is determined in accordance with protocols for administration of such agents alone, that is, not in conjunction with thrombolytic therapy. The effective dose will vary depending upon the potency of the antagonist and, in some cases, upon the route of administration. For example, BM 13,177 can be administered orally or parenterally at doses of 0.1 to 100 mg/kg, preferably about 0.1 to about 10 mg/kg. See, e.g. Stegmeier et al., Thromb. Res. 35: 379(1984) and Gresele et al., Lancet, May 5, 1984, 991.

Plasminogen activators such as tPA, SK and UK, when used alone in thrombolytic therapy, are administered parenterally, e.g. intraarterially, intracoronarily, intramuscularly or, preferably, intravenously. tPA is typically infused intravenously in amounts of about 80 to about 150 mg per patient over a period of 0.25 to 6 hours. SK can be fused intravenously in amounts of about $5 \times 10^4$ to $3 \times 10^6$ IU per patient over a similar period. UK can also be infused intravenously in amounts of about 40 to about 100 mg per patient over a similar period. Data generated in development of this invention indicate that the amount of tPA effective to lyse a thrombus in adjunct therapy with an inhibitor of thromboxane-induced platelet aggregation is reduced to at least about 1/2 to as much as about 1/10 to 1/100 the effective dose of tPA in an absence of the thromboxane synthase inhibition. Thus, a useful dose of tPA in the method of the invention is about 1 to about 75 mg per adult patient, preferably about 2 to about 30 mg per adult patient, although use of higher doses such as are employed in the case of tPA alone, e.g. up to 150 mg/kg, is not precluded. It also appears that the period of infusion can be lessened to about 5 minutes to 3 hours, preferably about 15 to about 40 minutes, although the normal treatment time is also not precluded. Similar reductions in the dosage and/or time of infusion can be obtained using other plasminogen activators.

The pharmaceutical composition of the invention is designed for convenient co-administration, that is, simultaneous administration, of the antagonist and of the plasminogen activator. Such composition is a formulation for parenteral administration which comprises an amount of a thromboxane receptor antagonist effective to inhibit such platelet aggregation at the site of the occlusion during the period of thrombolytic therapy and an amount of the plasminogen activator effective to lyse a target thrombotic occlusion, as well as a diluent or other carrier suitable for parenteral administration. Thus, a typical pharmaceutical formulation of the invention can comprise 0.1 to 10 mg of BM 13,177, and 1 to 150 mg of tPA, 1 to 100 mg of UK or $5 \times 10^4$ to $3 \times 10^6$ IU of SK, per dosage unit in an aqueous, buffered, isotonic solution. A dosage unit is the amount administered to a patient, e.g. 1 to 1000 ml, depending on the drug concentration and length of infusion. The plasminogen activator can be formulated, with or without the antagonist. For example tPA can be formulated in a buffer such as sodium or ammonium acetate or adipate at pH 3.5 to 5.5. Additional excipients, such as, e.g. polyvinyl-pyrrolidone, gelatin, hydroxy cellulose, acacia, polyethylene glycol, mannitol and sodium chloride can also be added. Such composition can be lyophilized.

For convenient administration of the antagonist and the plasminogen activator at the same or different times, a kit is prepared comprising, in a single container such as a box, carton or other container, individual bottles, bag, vials or other containers each having an effective amount of the antagonist for oral or parenteral administration, as described above, and an effective amount of the plasminogen activator for parenteral administration, as described above. Such kit can comprise, for example, both pharmaceutical agents in separate containers, optionally as lyophilized plugs, and containers of solutions for reconstitution.

When both agents are provided in solution form, they can be contained in an infusion/injection system for simultaneous administration or in a tandem arrangement, e.g. having the platelet aggregation inhibitor in an i.v. injectable form linked in series, via tubing, to the plasminogen activator in a second infusion bag. Using such system, a patient can receive an initial bolus-type injection of the antagonist followed by an infusion of the plasminogen activator.

The following examples demonstrate utility of the invention and the advantages of the present invention over available thrombolytic therapies. The Examples are illustrative and not limiting.

## Example 1

Twenty-four mongrel dogs of either sex (9 to 18 kg) were anesthetized with pentobarbital sodium (30 mg/kg i.v., intubated and ventilated with room air at a tidal volume of 20 ml/kg and a frequency of 12 inflations/minutes (Harvard respirator, Harvard Apparatus, S. Natick, Massachusetts.) The right femoral artery and vein were exposed, and catheters were inserted for monitoring arterial blood pressure (Stratham P23 ID pressure transducer,) and administration of drugs, respectively. The left external jugular vein and the left saphenous vein were also exposed and catheters were inserted for continuous infusion of 5% dextrose in saline and infusion of drugs, respectively. A left thoractomy was performed in the fifth intercostal space, and the heart was suspended in a pericardial cradle. A 20 mm section of the left circumflex coronary artery was isolated proximal to the first obtuse marginal branch and instrumented proximal to distal as follows: electromagnetic flow probe (Model 501, Carolina Medical Electronics, Inc., King, North Carolina), stimulation electrode, adjustable mechanical occluder (Goldblatt et al., J. Exp. Med. 59:347 (1934) and a silk snare. The stimulation electrode was constructed from a 26-gauge hypodermic needle tip attached to a 30-gauge TEFLON fluropolymer-insulated silver coated copper wire. The mechanical occluder was constructed of stainless steel with a stainless steel screw (2 mm diameter) which could be manipulated to control vessel circumference. The occluder was adjusted to eliminate the reactive hyperemic response affecting resting coronary blood flow.

Continuous recordings of blood pressure and mean and phasic left circumflex coronary artery blood flow were obtained on a model 70 polygraph (Grass Instrument Co., Quincy, Massachusetts.) Zero flow and hyperemic flows were determined by occluding the circumflex coronary artery distal to the flow probe for 20 seconds. Thirty minutes past surgical preparation, a 150 mA continuous anodal direct currentstimulation was initiated and maintained until the left circumflex (LCX) coronary artery blood flow decreased to and remained at 0 ml/minute for 30 minutes.

Direct electrical stimulation was delivered from a Grass constant current unit (Model CCUIA), a Grass stimulus isolation unit (Model S1U5B) and a Grass stimulator (Model 548) to the intraluminal coronary artery electrode (Grass). Sodium heparin 300 U/kg, i.v. was administered at 30 minutes post LCX occlusion and every hour thereafter at 100 U/kg, i.v. Dogs were assigned randomly to one of two groups. In Group I, placebo for BM 13,177 was administered for 120 minutes, at 45 minutes post LCX occlusion. The animals in Group II, at 45 minutes post LCX occlusion, received BM 13,177 at a bolus dose 10 mg/kg i.v. followed by 10 mg/kg/minutes i.v. for 120 minutes. At 15 minutes post-vehicle or active drug, tPA (from recombinant Chinese hamster ovary cells) was infused at 10 mg/kg/minutes i.v. for up to 90 minutes, if necessary to elicit reperfusion. tPA infusion in both Groups I and II was allowed to run for at least 30 minutes, regardless of reperfusion. If reperfusion occurred before 30 minutes, the infusion was terminated at 30 minutes whereas when reperfusion occurred beyond 30 minutes, the infusion was terminated at that point. Reperfusion was assessed as at least a 50% return of coronary blood flow compared to the control coronary blood flow. Furthermore, this level of blood flow had to be maintained for at least 5 minutes before termination of the tPA infusion.

Upon electrical stimulation of the circumflex coronary electrode, complete occlusion occurred at 37 ± 4 minutes for Group I and at 32 ± 6 minutes for Group II (Table 1). Thus, Group I and II animals did not differ in body weight, resting coronary blood flow or time to electrically-induced thrombotic occlusion.

tPA-induced coronary thrombolysis was achieved at a dose of 10 mg/kg/minutes, i.v. in all 26 animals studied from both Groups I and II. However, the time to thrombolysis differed dramatically between Group I and Group II animals (Table 1). Group I reperfusion occurred at 32 ± 6 minutes whereas Group II reperfusion occurred at 11 ± 2 minutes (p<0.05). Thus, administration of BM 13,177 at a bolus dose of 10 mg/kg followed by an infusion of 10 mg/kg/hr, 15 minutes before administration of tPA, resulted in a significantly shorter time to restoration of coronary blood flow.

Table 1

| Characteristic of Coronary Blood Flow in Response to Thrombosis, Thrombolysis and Rethrombosis in the Dog | | |
|---|---|---|
| | Group I | Group II |
| | (n = 15) | (n = 11) |
| Time to occlusion (min) | 37 ± 4 | 32 ± 6 |
| Time to Reperfusion (min) | 32 ± 6 | 11 ± 2 |
| Time to Reocclusion (min) | 39 ± 12 | 35 ± 0 |
| Number Reoccluded[1] | 12/15 | 1/9[*2] |

*p<0.05 vs. control
[1]Reocclusion defined as zero coronary blood flow after tPA of permanent or non-permanent nature.
[2]Only 9 dogs are included in reocclusion data as 2 of the 11 dogs fibrillated at 27 and 30 minutes after thrombolysis and were excluded.

Upon termination of the tPA infusion, reocclusion occurred in 12 of 15 Group I animals (80%) and in only 1 of 9 (Group II) animals (11%). Two animals in Group II were excluded from the reocclusion data as they fibrillated 27 and 30 minutes after termination of the tPA infusion. However, these animals had coronary blood flows of 10 and 12 ml/minutes, respectively just before fibrillation but suffered from severe tPA-induced reperfusion arrhythmia. Reocclusion was assessed as zero blood flow after termination of the tPA infusion of either a permanent or non-permanent nature. Most animals who reoccluded, exhibited cyclical variations in coronary blood flow after tPA such that flow reached zero and then spontaneously was restored throughout the 2 hour observation period after termination of tPA. Complete permanent occlusion occurred in only 2 of 12 dogs in Group I and in 1 of 1 in Group II. The time to the first occlusive zero after tPA was 39 ± 12 minutes in Group I and 35 ± 0 minutes in Group II (Table 1).

Control coronary blood flow was similar in both groups. Reperfusion coronary blood flow was greater for Group II although not significantly different from Group I. However, coronary blood flow assessed at termination of the experiment was significantly greater for Group II compared to Group I animals (p<0.05).

Example 2

Blood was withdrawn from the carotid artery of a dog into a plastic syringe containing one part 3.8% trisodium citrate to nine parts blood. Platelet rich plasma (PRP) was obtained by centrifugation of this mixture at 200 x g for 10 minutes at room temperature. Platelet count was adjusted to 300,000/mm³. All aggregation studies were performed in a Chrono-Log aggregometer (Chrono-Log Corporation, Havertown, Pennsylvania) using 0.3 ml PRP in a siliconized cuvette stirred at 1100 rpm. PRP was prewarmed for two minutes 37°C before addition of agonist. Agonists of different molar concentrations were added to the cuvette and the change in light transmission was measured to the point where the tracing reached a plateau. Platelet function was assessed in PRP via light transmission aggregometry after i.v. BM 13.177 and compared to that in PAP preparations without BM 13.177.

Specifically, the agonists were U-46619 (Upjohn Diagnostics, Kalamazoo, Michigan) (1.0 mM) and collagen (Hormon-Chemie, Munich, Federal Republic of Germany) (5N0 mg/ml). These were added to test PRP preparations at 5 minutes and at 2 hours after BN 13.177 treatment.

BM 13.177 (10 mg/kg plus 10 mg/kg/hr) inhibited fully both U-46619 and collagen-induced aggregation at 30 minutes and 2 hour post-initial administration.

Examples 1 and 2 thus demonstrate the thrombolytic therapy employing a plasminogen activator and a thromboxane receptor antagonists produces more rapid fibrinolysis and reperfusion than such therapy with a plasminogen activator along.

**Claims**

1. A thrombolytic composition for parenteral administration to effect thrombolysis and reperfusion of a thrombotic occlusion in a mammal which comprises a plasminogen activator, a thromboxane receptor antagonist and a pharmaceutically acceptable carrier.

2. The composition of claim 1 wherein the plasminogen activator has affinity for fibrin.

3. The composition of claim 2 wherein the plasminogen activator is tPA, UK or SK.

4. The composition of claim 3 wherein the inhibitor is BM 13,177 or BM 13,505.

5. The composition of claim 1 wherein the plasminogen activator is tPA in an amount effective to reperfuse the occluded vessel and the antagonist is in an amount effective to inhibit reocclusion following reperfusion.

6. The composition of claim 5 comprising 0.1 to 100 mg of BM 13,177 and 1 to 150 mg of tPA per dosage unit.

7. A kit for use in effecting thrombolysis and reperfusion of a thrombotic occlusion in a mammal which comprises, in separate containers, a plasminogen activator and a thromboxane receptor antagonist.

8. The kit of claim 7 wherein the plasminogen activator has affinity for fibrin.

9. The kit of claim 7 wherein the plasminogen activator is tPA, UK or SK.

10. The kit of claim 8 wherein the antagonist is BM 13,177 or BM 13,505.

11. The kit of claim 7 wherein the plasminogen activator is tPA in an amount effective to reperfuse the occluded vessel and the antagonist is in an amount effective to inhibit reocclusion following reperfusion.

12. The kit of claim 11 comprising 0.1 to 100 mg of BM 13,177 and 1 to 150 mg of tPA per dosage unit.

13. A process for preparing a thrombolytic composition according to claim 1 which comprises bringing into association a plasminogen activator, a thromboxane receptor antagonist and a pharmaceutically acceptable carrier.

Claims for the following Contracting States : GR, ES.

1. A process for preparing a thrombolytic composition for parenteral administration to effect thrombolysis and reperfusion of a thrombotic occlusion in a mammal which comprises bringing into association a plasminogen activator, in an amount effective to lyse the clot, and a thromboxane receptor antagonist in an amount effective to inhibit reocclusion and a pharmaceutically acceptable carrier.

2. The process of claim 1 wherein the plasminogen activator has affinity for fibrin.

3. The process of claim 2 wherein the plasminogen activator is tPA, UK or SK.

4. The process of claim 3 wherein the antagonist is BM 13,177 or BM 13,505.

5. The process of claim 4 wherein the plasminogen activator is tPA or UK.

6. The process of claim 1 wherein the plasminogen activator is tPA in an amount effective to reperfuse the occluded vessel and the antagonist is in an amount effective to inhibit reocclusion following reperfusion.

7. The process of claim 6 comprising 0.1 to 100 mg of BM 13,177 and 1 to 150 mg of tPA per dosage unit.

| | European Patent Office | EUROPEAN SEARCH REPORT | Application Number |
|---|---|---|---|
| | | | EP 89 12 0582 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | CHEMICAL ABSTRACTS, vol. 101, no. 19, 5th November 1984, page 34, abstract no. 163411c, Columbus, Ohio, US; K. STEGMEIER et al.: "The pharmacological profile of the thromboxane A2 antagonist BM 13.177. A new anti-platelet and anti-thrombotic drug", & THROMB. RES. 1984, 35(4), 379-95 * Abstract * | 1-13 | A 61 K 45/06 A 61 K 37/54 // (A 61 K 37/54 A 61 K 31:19 ) |
| A | CHEMICAL ABSTRACTS, vol. 104, no. 13, 31st March 1986, page 46, abstract no. 102194z, Columbus, Ohio, US; H. DARIUS et al.: "Antiaggregatory effects of thromboxane receptor antagonists in vivo", & THROMB. RES. 1985, 40(5), 663-75 * Abstract * | 1-13 | |
| | | | TECHNICAL FIELDS SEARCHED (Int. Cl.5) A 61 K |

The present search report has been drawn up for all claims

| Place of search | Date of completion of the search | Examiner |
|---|---|---|
| THE HAGUE | 08-02-1990 | BRINKMANN C. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)